# EUROPEAN PATENT SPECIFICATION

(11) **EP 2 189 161 B1**
(45) Date of publication and mention of the grant of the patent: **08.08.2012**
(21) Application number: 08830964.6
(22) Date of filing: 08.09.2008
(51) Int. Cl.: A61K 31/7072, A61P 31/12, A61P 35/00, C12Q 1/02, C12Q 1/18

(54) **MEDICINAL AGENT FOR DISEASE ASSOCIATED WITH EPSTEIN-BARR VIRUS, AND METHOD FOR SCREENING OF THE MEDICINAL AGENT**
ARZNEIMITTEL FÜR MIT EPSTEIN-BARR-VIRUS ASSOZIIERTE ERKRANKUNG UND PRÜFVERFAHREN FÜR DAS ARZNEIMITTEL
AGENT THÉRAPEUTIQUE DESTINÉ À UNE MALADIE ASSOCIÉE AU VIRUS D'EPSTEIN-BARR ET PROCÉDÉ DE CRIBLAGE DE CE DERNIER

(30) Priority: 10.09.2007 JP 2007233552
(43) Date of publication of application: 26.05.2010
(73) Proprietor: YAMASA CORPORATION, Choshi-shi Chiba 288-0056 (JP); Kyoto University, Kyoto-shi Kyoto 606-8501 (JP)
(72) Inventor: KODAMA, Eiichi, Kyoto-shi, Kyoto 606-8507 (JP); MATSUOKA, Masao, Kyoto-shi, Kyoto 606-8507 (JP); ASHIDA, Noriyuki, Choshi-shi Chiba 288-0056 (JP)
(74) Representative: Blodig, Wolfgang
(86) International application number: PCT/JP2008/066175
(87) International publication number: WO 2009/034945

(56) References cited:
- EP-A1- 0 421 777
- WO-A1-95/20595
- JP-A- 05 505 791
- JP-A- 09 508 394
- JP-A- 2005 528 334
- US-A1- 2003 176 392
- MACHIDA H ET AL: "ANTI-HERPESVIRUS ACTIVITY PROFILE OF 4'-THIOARABINOFURANOSYL PURINE AND URACIL NUCLEOSIDES AND ACTIVITY OF 1-BETA-D-2'-FLUORO-4'-THIOARAB INOFURANOSYL GUANINE AND 2,6-DIAMINOPURINE AGAINST CLINICAL ISOLATES OF HUMAN CYTOMEGALOVIRUS", ANTIVIRAL RESEARCH, ELSEVIER BV, NL, vol. 39, 1 January 1998 (1998-01-01), pages 129-137, XP002950789, ISSN: 0166-3542, DOI: 10.1016/S0166-3542(98)00042-4
- YOSHIMURA Y ET AL: "Synthesis and biological activities of 2'-deoxy-2'-fluoro-4'-thioarab inofuranosylpyrimidine and -purine nucleosides.", BIOORGANIC & MEDICINAL CHEMISTRY JUL 2000 LNKD- PUBMED:10976503, vol. 8, no. 7, July 2000 (2000-07), pages 1545-1558, XP002660230, ISSN: 0968-0896

## Description

### TECHNICAL FIELD

The present invention relates to an anti-Epstein-Barr virus agent and a medicinal agent for a disease associated with an EB virus.

### BACKGROUND ART

An EB virus is considered responsible for various tumors such as B lymphoma, nasopharyngeal cancer and gastric cancer though the incidence rate due to it is not high. As a treatment for such a tumor in an inoperable case, an ordinary anticancer agent is administered, but it is not always an effective treatment. Ganciclovir, an antiviral agent has been reported to be suitable as a therapeutic agent for the tumors (non-patent document 1), but it is not sufficiently effective and hence has not yet been put to practical use.
In addition, a gene therapy for the disease with use of thymidine phosphorylation enzyme (thymidine kinase: TK), which is derived from EB virus related to herpes simplex virus has also been tried in laboratory, but it has not yet been applied to patients.

On the other hand, 1-(2-fluoro-4-thio-β-D-arabinofuranosyl)-5-methyluracil is known as a compound having a low antiviral activity against herpesviruses (non-patent document 2, patent document 1 and patent document 2), but the antiviral activity of the compound against an EB virus and its effect on tumors caused by the EB virus have not been known at all.
Non-patent document 1: Antimicrob. Agents Chemother 2001; 45(7): 2082-91
Non-patent document 2: Antiviral Res. 1998; 39(2): 129-37
Patent document 1: International Publication No. WO91/04982
Patent document 2: JP-A-10-87687

EP 0 421 777 A1 relates to antiviral nucleosides and mentions 1-(2-fluoro-4-thio-β-D-arabinofuranosyl)-5-methyl uracil. WO 95/20595 relates to L-nucleosides for the treatment of EB virus. The compound "FMAU" (1-(2-deoxy-2-fluoro-β-D-arabinofuranosyl)-5-methyl uracil) used as a comparative compound in the present invention is also mentioned. Machida et al., Antiviral Research 39 (1998) 129-137 relates to the anti-herpesvirus activity of 4-thioarabinofuranosyl uracil nucleosides. US 2003/0176392 relates to the treatment of EB virus infections using 5-substituted uracil nucleosides.

### DISCLOSURE OF THE INVENTION

### Problem to be Solved by the Invention

Therefore, the present invention is intended to find an anti-EB virus agent and a medicinal agent having a marked effect on a disease associated with an EB virus.

### Means for Solving the Problem

No effective screening method for finding a medicinal agent for a disease associated with an EB virus has been reported. Therefore, the present inventors attempted to establish an effective screening method at first and find a medicinal agent having a marked effect on a disease associated with an EB virus by the use of the screening method. As a result, by utilizing TK derived from an EB virus which is present in tumor cells, the present inventors have established a method for screening a medicinal agent which is specifically phosphorylated by the TK to exhibit toxicity against the cells. As a result of screening of various compounds by the above-mentioned screening method, the present inventors have found that 1-(2-fluoro-4-thio-β-D-arabinofuranosyl)-5-methyluracil has an anti-EB virus activity and a marked effect on a disease associated with an EB virus.

Therefore, the present invention has been accomplished on the basis of the above finding and has the following aspects [1] to [3].
[1] 1-(2-fluoro-4-thio-β-D-arabinofuranosyl)-5-methyluracil, a salt thereof, or a hydrate or solvate of either of them for use in a method for treatment of a disease associated with Epstein-Barr (EB) virus.
[2] A prophylactic or therapeutic agent which comprises 1-(2-fluoro-4-thio-β-D-arabinofuranosyl)-5-methyluracil, a salt thereof, or a hydrate or solvate of either of them as an active ingredient for use in a method for treatment of a disease associated with an EB virus.
[3] A prophylactic or therapeutic agent according to the above item [2], wherein the disease associated with an EB virus is a tumor caused by the EB virus or an acute or chronic EB virus infectious disease.

Also described is a method for screening a medicinal agent having an anti-EB virus activity which comprises introducing each of a plasmid capable of expressing EB virus-TK gene and a plasmid capable of expressing human-TK gene into a thymidine kinase (TK)-defect cell to produce two types of cells respectively having the plasmids introduced therein; and screening a medicinal agent which has cytotoxicity against the cell having the plasmid capable of expressing EB virus-TK gene introduced therein but has no cytotoxicity against the cell having the plasmid capable of expressing human-TK gene introduced therein, by the use of the above-mentioned two types of cells.

### Advantages of the Invention

The medicinal agent of the present invention has cytotoxicity only against cells in which EB virus-TK gene has been expressed, and it has no cyotoxicity against cells in which human-TK gene has been expressed. Therefore, the medicinal agent may be expected to have anti-EB virus effect, prophylactic and therapeutic effects on a disease associated with an EB virus, and only reduced side effects. Thus, the medicinal agent is useful as a prophylactic or therapeutic agent for a disease caused by an EB virus, such as an acute or chronic EB virus infectious disease or a malignant tumor caused by the EB virus.

In the screening method, the utilization of TK derived from an EB virus makes it possible to screen a medicinal agent which is specifically phosphorylated by the TK to exhibit cytotoxicity against cells. Therefore, the screening method is useful as a method for screening a medicinal agent capable of exhibiting anti-EB virus activity.

### BEST MODE FOR CARRYING OUT THE INVENTION

The screening method used for the present invention is explained below at first, and then the medicinal agent of the present invention selected by the screening method is explained.

### (1) The screening method used for the present invention

As described above, in the screening method, each of a plasmid capable of expressing EB virus-TK gene and a plasmid capable of expressing human-TK gene is introduced into a TK-defect cell to produce two types of cells respectively having the plasmids introduced therein, and by the use of the two types of cells. A medicinal agent is screened which has cytotoxicity against the cell having the plasmid capable of expressing EB virus-TK gene introduced therein but has no cytotoxicity against the cell having the plasmid capable of expressing human-TK gene introduced therein,

As the TK-defect cell, a TK-defect human tumor cell is preferable. Examples thereof include a TK-defect human osteosarcoma cell (143B). Such a cell is available from ATCC or the like, and the 143B cell mentioned above has been registered as ATCC CRL-8303.

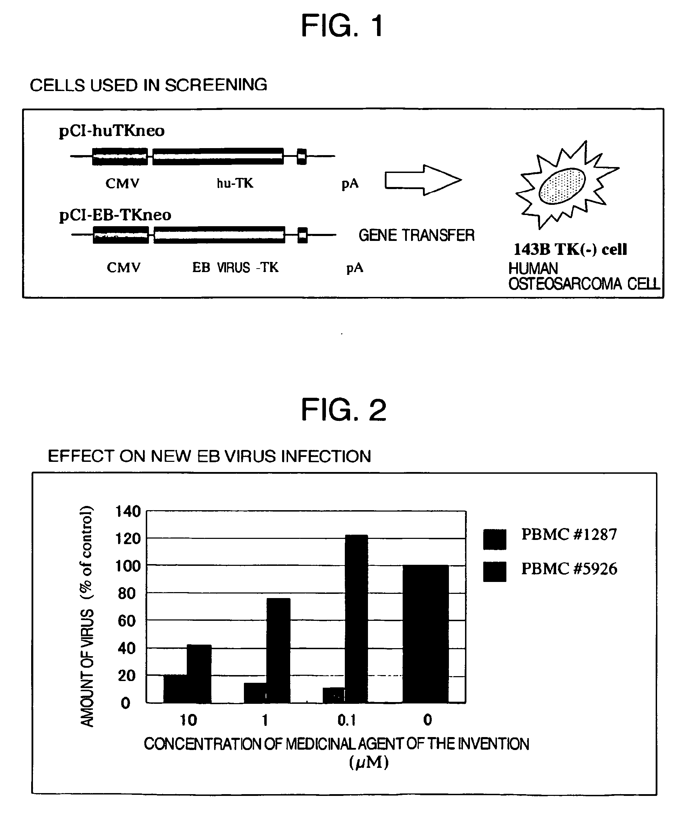

Examples of the plasmid vector capable of expressing EB virus-TK gene or human-TK gene include a plasmid which, as shown in Fig. 1, has suitable antibiotic resistance for selection and a gene coding for EB virus-TK or human-TK which is connected in an expressible state downstream to a promoter.

The gene coding for EB virus-TK or human-TK has already been cloned and its base sequence may be looked up by the use of the data base of National Center for Biotechnology Information (NCBI). Therefore, the gene coding for EB virus-TK or human-TK may be prepared by a conventional method on the basis of the data base information of NCBI.

Next, the TK gene prepared above is connected to a plasmid having suitable antibiotic resistance and, for example, a promoter and a terminator derived from cytomegalovirus (CMV), by a conventional method. Specifically, the gene coding for EB virus-TK or human-TK is connected to a multicloning site downstream to the promoter of pCI-neo Mammalian Expression Vector, a plasmid available from Promega Inc. by a conventional method so as to enable the expression.

The above-mentioned TK-defect cell is transfected with the plasmid prepared. The gene transfection may be carried out by a conventional method (Proc. Natl. Acad. Sci. USA 1987 Nov; 84(21): 7413-7).

Using the thus prepared two types of cells having the EB virus-TK gene and human-TK gene, respectively, transfected thereto, a medicinal agent is screened which has cytotoxicity against the cell having EB virus-TK gene introduced therein but has no cytotoxicity against the cell having human-TK gene introduced therein, whereby the medicinal agent having an anti-EB virus activity may be screened. In particular, it is possible to express the selective toxicity of a test medicinal agent in terms of a coefficient by measuring a concentration (CC₅₀) of the test medicinal agent at which the cell viability of each of the cells is decreased to 50%, and calculating the ratio between the thus measured concentrations as a selection index (SI).

### (2) The medicinal agent of the present invention

The active ingredient of the medicinal agent of the present invention is 1-(2-deoxy-2-fluoro-4-thio-β-D-arabinofuranosyl)-5-methyluracil (another name: 1-(2-deoxy-2-fluoro-4-thio-β-D-arabinofuranosyl)thymine). This compound is a well-known compound and may be prepared by a method known in literature or a modification thereof (see non-patent document 2, patent document 1, patent document 2 or the like). This compound may be in the form of a salt, hydrate or solvate. Examples of such a salt include acid adducts such as hydrochloride and sulfate. Examples of the hydrate or solvate include compounds produced by adhesion of 0.1 to 3.0 molecules of water or a solvent to a molecule of the above-mentioned compound or salt thereof. The above-mentioned compound also includes its various isomers such as its tautomers.

The medicinal agent of the present invention is effective as an anti-EB virus agent and a prophylactic or therapeutic agent for a disease associated with an EB virus. Examples of the disease associated with an EB virus include tumors caused by the EB virus and acute or chronic EB virus infectious diseases. Examples of the tumors caused by the EB virus include B lymphoma, nasopharyngeal cancer and gastric cancer.
The dose of the medicinal agent of the present invention is varied depending on the age, body weight, disease of a patient, the seriousness of the disease of the patient, tolerance for a drug, an administration route and the like, and is properly determined by considering these conditions synthetically. The dose is usually chosen in the range of 0.001 to 1000 mg/kg body weight, preferably 0.001 to 100 mg/kg body weight, per day. The medicinal agent is administered in one portion or several portions. As to the administration route, the medicinal agent may be administered by any of oral, non-oral, enteral and topical administrations and the like.

For formulating the above-mentioned compound into a pharmaceutical preparation, conventional additives such as a carrier for formulation, excipient and the like may be used. Examples of the carrier include solid carriers such as lactose, kaolin, sucrose, crystalline cellulose, corn starch, talc, agar, pectin, stearic acid, magnesium stearate, lecithin, sodium chloride ; and liquid carriers such as glycerol, peanut oil, polyvinylpyrrolidones, olive oil, ethanol, benzyl alcohol, propylene glycol, water. The pharmaceutical form may be any form. For example, when the solid carrier is used, examples of the pharmaceutical form include tablets, powders, granules, capsules, suppositories and troches. When the liquid carrier is used, examples of the pharmaceutical form include syrups, emulsions, soft gelatin capsules, creams, gels, pastes, sprays and injections.

### EXAMPLES

The present invention will be concretely explained with reference to a working example .

### Example

### (1) Establishment of a cell strain

Fig. 1 shows an outline of a method for screening a medicinal agent which is specifically phosphorylated by EB virus-TK to exhibit cytotoxicity against cells.

At first, a primer was designed on the basis of the data base information of NCBI, followed by extracting DNA from B95a cells (J. Virol. 1990; 64(2): 700-5), and the TK gene of an EB virus was amplified by PCR method and cloned in the multicloning site of an expression plasmid vector pCI-neo (pCI-EB-TKneo). Similarly, human-TK gene was also amplified by PCR method and cloned (pCI-huTKneo). Whether the cloned gene fragment was a desired gene or not was judged by determining the base sequence.

Then, TK-defect human osteosarcoma cells (143B) was transfected by lipofection method (Proc. Natl. Acad. Sci. USA 1987 Nov; 84(21): 7413-7) with the plasmid vector capable of expressing EB virus-TK gene or the plasmid vector capable of expressing human-TK gene, namely, pCI-EB-TKneo or pCI-huTKneo, and cells having each plasmid introduced therein were selected by the use of neomycin.

Using these two types of cells, i.e., the cell having EB virus-TK gene introduced therein (143B/EB virus-TK) and the cell having human-TK gene introduced therein (143B/hu-TK), a medicinal agent was screened which had cytotoxicity against the cell having EB virus-TK gene introduced therein but had no cytotoxicity against the cell having human-TK gene introduced therein.

### (2) Determination of drug sensitivity

The drug sensitivity of 143B/EB virus-TK, 143B/hu-TK, 143B TK(-) cells and EB virus persistent infection cells was determined on the basis of the cytotoxicity of each drug. That is, cells (143B/EB virus-TK, 143B/hu-TK and 143B TK(-) cells: 5 x 10⁴ cells/100 µl; EB virus persistent infection cells: 20 x 10⁴ cells/100 µl) were added to 96-well flat-bottom culture plates containing 100 µl of various concentrations of each drug. After 5 days of culture, the number of surviving cells was determined by MTT [3-(4,5-dimethylthiazol-2-yl)-2,5-diphenyltetrazolium bromide] assay. The cytotoxicity of the test drugs is expressed in terms of a concentration (CC₅₀) at which the cell viability was decreased to 50%.

### (3) Effect of drugs

By MTT assay, there was judged whether or not nucleic acid derivatives whose phosphorylation by EB virus-TK has been reported (azidothymidine (AZT), 5-fluoro-2'-deoxyuridine (5FdU), acyclovir (ACV), 1-β-D-arabinofuranosyl-5-bromovinyluracil (BVaraU), ganciclovir (GCV) and 5-bromovinyl-2'-deoxyuridine (BVDU)) had selective toxicity against 143B/EB virus-TK in which EB virus-TK gene had been expressed. In the same manner as above, there were investigated the following nucleic acid derivatives having a structure similar to that of the compound according to the present invention: 1-(2-deoxy-2-fluoro-β-D-arabinofuranosyl)-5-methyl uracil (FMAU), 1-(C-cyano-2-deoxyribofuranosyl)thymine (1'-CN-dT), β-D-2-methylene-4-thio-β-D-erythropentofuranosyl)thymine (4'-S-DMDT), 1-(2-deoxy-4-thio-β-D-arabinofuranosyl)thymine (4'-S-araT) and 1-(2-deoxy-2-fluoro-4-thio-β-D-arabinofuranosyl)cytosine (4'-S-FMAC).

As a result, as shown in Table 1, no drug having selective toxicity against 143B/EB virus-TK cells could be found except the medicinal agent of the present invention. In addition, as a result of screening of 130 nucleic acid derivatives other than the above-mentioned nucleic acid derivatives in the same manner as above, no drug having a marked effect could be found.

### (4) Effect of the medicinal agent of the present invention

In the same manner as in the above item (3), the cytotoxicity of the medicinal agent of the present invention against 143B/EB virus-TK cells and 143B/hu-TK (human-TK) cells was investigated by MTT assay to find that as shown in Table 1, the medicinal agent had the highest selection index (SI: more than 2700-fold) for 143B/EB virus-TK cells but had no cytotoxicity against 143B/hu-TK cells even at 300 µM.

**[Table 1]**

| Table 1: Cytotoxic effect of various drugs | | | |
|---|---|---|---|
| | CC₅₀ (µM) | | |
| Drug | 143B/EB Virus-TK | SI | 143B/hu-TK |
| AZT | >100 | | >100 |
| 5FdU | 0.40 | 0.7 | 0.27 |
| BvaraU | >200 | | >200 |
| BVDU | 122 | >2.5 | >300 |
| GCV | >100 | | >100 |
| ACV | >100 | | >100 |
| FMAU | 320 | 0.9 | 290 |
| 1'-CN-dT | >100 | | >100 |
| 4'-S-DMDT | >100 | | >100 |
| 4'-S-araT | >100 | | 100 |
| 4'-S-FMAC | 0.066 | 0.6 | 0.040 |
| Medicinal agent of the invention | 0.11 | >2700 | >300 |

### (5) Effect of the medicinal agent of the present invention on EB virus infection cancer cells

The cytotoxic effect of the medicinal agent of the present invention on EB virus infection cancer cells such as NC37 cells was investigated by MTT assay. As a result, it was found that as shown in Table 2, the medicinal agent of the present invention was more than 10 times as effective as an existing drug GCV against NC37 cells in which EB virus-TK gene had been expressed. However, neither of them was effective in cells in which EB virus-TK gene had not been expressed.

**[Table 2]**

| Table 2: Cytotoxic effect on EB virus infection cancer cells | | | |
|---|---|---|---|
| Cell | Expression of TK of TK | Medicinal agent of the invention µM | GCV µM |
| B95a | + | 2.2 | 36 |
| NC37 | + | 2.1 | 27 |
| Raji | - | 22 | 55 |
| Namalwa | - | >100 | >100 |
| HS-Sultan | - | >100 | >100 |

### (6) Effect on acute infection

B lymphocytes were separated from the blood of a healthy person. The B lymphocytes were infected with an EB virus in the presence of the medicinal agent of the present invention and cultured for 2 weeks. Thereafter, the infected B cells were recovered and then washed, followed by extraction of DNA. The amount of EB virus DNA in the B lymphocytes was determined by real-time PCR and taken as a virus copy number. Fig. 2 shows the results obtained in two typical cases (PBMC#1287 and PBMC#5926). It was proved that as shown in Fig. 2, the medicinal agent of the present invention reduces the amount of EB virus DNA in the B lymphocytes and inhibits new infection.

### (7) Effect on cells with a chronic active EB virus infectious disease

A chronic active EB virus infectious disease is an unfavorable infectious disease caused by infection of not B cells but natural killer T cells (NKT cells) with an EB virus. As such a chronic infection cell strain, KAI3 cells (Clin. Exp. Immunol. 1999 Mar; 115(3): 385-92) were purchased from Health Science Research Resources Bank, and the effect of the medicinal agent of the present invention on them was investigated by MTT assay. GCV was used as a control agent. As a result, it was found that as shown in Table 3, the medicinal agent of the present invention was clearly more effective than GCV.

Thus, it was proved that the medicinal agent of the present invention is more than 10 times as effective as GCV whose effect has been reported. Therefore, it is considered that the medicinal agent of the present invention has cytotoxicity only against cells in which EB virus-TK gene has been expressed, and that the medicinal agent can ablate EB virus-infected cells irrespective of whether the infection is acute or chronic.

**[Table 3]**

| Table 3: Effect on cells with a chronic active EB virus infectious disease | |
|---|---|
| Medicinal agent of the invention | 4.7 µM |
| GCV | 105 µM |

### INDUSTRIAL APPLICABILITY

The medicinal agent of the present invention is useful as a prophylactic or therapeutic agent for a disease caused by an EB virus, such as an acute or chronic EB virus infectious disease or a malignant tumor caused by the EB virus, which has only reduced side effects. The screening method of the present disclosure is useful as a method for screening a medicinal agent capable of exhibiting an anti-EB virus activity specifically.

### BRIEF DESCRIPTION OF THE DRAWINGS

[Fig. 1] A schematic view showing an outline of the screening method of the present disclosure.
[Fig. 2] A graph showing the effect of the medicinal agent of the present invention on the amount of EB virus DNA in B lymphocytes.

## Claims

1. 1-(2-deoxy-2-fluoro-4-thio-β-D-arabinofuranosyl)-5-methyluracil, a salt thereof, or a hydrate or solvate of either of them for use in a method for treatment of a disease associated with an Epstein-Barr (EB) virus.

2. The 1-(2-deoxy-2-fluoro-4-thio-β-D-arabinofuranosyl)-5-methyluracil, a salt thereof, or a hydrate or solvate of either of them for use according to claim 1, wherein the disease associated with an EB virus is an acute or chronic EB virus infectious disease or a tumor caused by the EB virus.

3. A prophylactic or therapeutic agent which comprises 1-(2-deoxy-2-fluoro-4-thio-β-D-arabinofuranosyl)-5-methyluracil, a salt thereof, or a hydrate or solvate of either of them as an active ingredient for use in a method for treatment of a disease associated with an Epstein-Barr (EB) virus.

4. The prophylactic or therapeutic agent for the use according to claim 3, wherein the disease associated with an EB virus is an acute or chronic EB virus infectious disease or a tumor caused by the EB virus.

## Patentansprüche

1. 1-(2-Deoxy-2-fluor-4-thio-β-D-arabinofuranosyl)-5-methyluracil, ein Salz davon, oder ein Hydrat oder Solvat davon, zur Verwendung in einem Verfahren zur Behandlung einer mit einem Epstein-Barr (EB) Virus assoziierten Erkrankung.

2. Das 1-(2-Deoxy-2-fluor-4-thio-β-D-arabinofuranosyl)-5-methyluracil, ein Salz davon, oder ein Hydrat oder Solvat davon, zur Verwendung nach Anspruch 1, wobei die mit einem EB Virsus assoziierte Erkrankung eine akute oder chronische EB Virusinfektionserkrankung oder ein von dem EB Virus hervorgerufener Tumor ist.

3. Prophylaktisches oder therapeutisches Mittel, das 1-(2-Deoxy-2-fluor-4-thio-β-D-arabinofuranosyl)-5-methyluracil, ein Salz davon oder ein Hydrat oder Solvat davon als wirksamen Bestandteil umfasst zur Verwendung in einem Verfahren zur Behandlung einer mit einem Epstein-Barr (EB) Virus assoziierten Erkrankung.

4. Das prophylaktische oder therapeutische Mittel zur Verwendung nach Anspruch 3, wobei die mit einem EB Virus assoziierte Erkrankung eine akute oder chronische EB Virusinfektionserkrankung oder ein von dem EB Virus hervorgerufener Tumor ist.

## Revendications

1. 1-(2-désoxy-2-fluoro-4-thio-β-D-arabinofuranosyl)-5-méthyluracil, un sel de celui-ci, ou un hydrate ou solvate de l'un d'entre eux, pour son utilisation dans une méthode de traitement d'une maladie associée à un virus d'Epstein-Barr (EB).

2. 1-(2-désoxy-2-fluoro-4-thio-β-D-arabinofuranosyl)-5-méthyluracil, un sel de celui-ci, ou un hydrate ou solvate de l'un d'entre eux, pour son utilisation selon la revendication 1, dans lequel la maladie associée à un virus EB est une maladie infectieuse par virus EB aiguë ou chronique ou une tumeur causée par le virus EB.

3. Agent prophylactique ou thérapeutique comprenant du 1-(2-désoxy-2-fluoro-4-thio-β-D-arabinofuranosyl)-5-méthyluracil, un sel de celui-ci, ou un hydrate ou solvate de l'un d'entre eux, comme ingrédient actif pour son utilisation dans une méthode de traitement d'une maladie associée à un virus d'Epstein-Barr (EB).

4. Agent prophylactique ou thérapeutique pour son utilisation selon la revendication 3, dans lequel la maladie associée à un virus EB est une maladie infectieuse par virus EB aiguë ou chronique ou une tumeur provoquée par le virus EB.
